# EUROPEAN PATENT APPLICATION

(11) **EP 1 769 801 A1**
(43) Date of publication of application: **04.04.2007**
(21) Application number: 05388079.5
(22) Date of filing: 28.09.2005
(51) Int. Cl.: A61K 35/74

(54) **Use of a fermented cereal composition for the treatment of IBD and IBS**

(71) Applicant: Israelsen, Hans, 3450 Allerod (DK)
(72) Inventor: Israelsen, Hans, 3450 Allerod (DK)
(74) Representative: Olsen, Lars Pallisgaard

(57) **Abstract**

The invention covers a novel treatment strategy that considerably improves conventional probiotic treatments of inflammatory bowel diseases, irritable bowel syndrome and other gastrointestinal disorders. Both probiotic microorganisms and the carrier of the probiotic microorganisms in form of a fermented cereal gruel are used as treatment effectors. The novel treatment strategy is capable of removing the symptoms of inflammatory bowel diseases regardless of a mild, moderate or severe stage of the disease.

## Description

### FIELD OF THE INVENTION

The present invention relates to the treatment and maintenance treatment of inflammatory bowel diseases and inflammatory bowel syndrome. In particular, it concerns the use of probiotic bacteria and a carrier as treatment effectors in a novel treatment concept.

### BACKGROUND OF THE INVENTION

Ulcerative colitis (UC) and Crohn's Disease (CD) are inflammatory bowel diseases (IBD) characterized by chronic inflammation in the intestine. UC occurs in the colon while CD may be present in the entire gastrointestinal (GI) tract. The clinical symptoms are diarrhea, abdominal pain, occasional rectal bleeding, weight loss, tiredness and sometimes fever. Although occurring at any age, IBD is most common in teenagers and young adults, which consequently may suffer from delayed development and stunted growth. The frequency of the disease is similar to type 1 diabetes in Europe and the USA. The clinical course of IBD varies considerably. Patients with mild to moderate symptoms may be treated without hospitalization. However, 10-15 % of patients experience a severe course of the disease, which in many cases is followed by surgery.

IBD is treated medically by reducing the inflammation and thereby controlling the gastrointestinal symptoms. However, there is currently no medical cure for IBD. Coloectomy may eliminate UC but reduces life quality and increases the risk of complications. The available medical treatments include the use of 5-aminosalicylic acid (5 ASA), corticosteroids and immunomodulatory medicaments. Prolonged treatment of mild to moderate IBD symptoms is usually carried out using 5 ASA while corticosteroids and immunomodulatory medicaments are used to treat severe symptoms. Diarrhea or abdominal pain appear as side effects of 5 ASA whereas long term use of corticosteroids frequently shows serious side effects including reduction in bone mass, infection, diabetes, muscle wasting and psychiatric disturbances. Immonomodulatory medicaments suppress the immune system, which controls the IBD symptoms. However, the resulting immuno-compromised state leaves the patient susceptible to many diseases.

IBD seems to be a result of an uncontrolled cascade in the immune response. The successful treatment of CD patients with antibodies against the pro-inflammatory cytokine TNFα supports this assumption (Rutgeerts et al. 2004 Gastroenterology 126:1593-610).

However, a prolonged antibody treatment will result in a general lowing of the TNFα level, which eventually will lead to susceptibility to other diseases.

The reason for the chronic uncontrolled immune response in IBD has not been established. However, both genetic dispositions and the composition of the microbial flora residing in the intestinal tract of the patient are putative causes. Recently it has been reported that several hundred genes may be involved in a genetic disposition for IBD (Costello et al. 2005 PLoS Med 2(8): e199), which makes the development of a successful treatments through genetic strategies extremely difficult. Although it was found that Helicobacter pylori is the cause for peptic ulcer disease, no specific pathogens have been found to be the cause of IBD. However, it is generally believed that the commensal microorganisms in the GI tract are key factors in the exaggerated immune response in IBD patients (Schultz et al. 2003 Dig. Dis. 21: 105-128). In vitro tests have shown that immune competent cells react differently upon contact to different bacteria (Christensen et al. 2002 J Immunol. 168:171-8). In general, microorganisms can be divided into two groups inducing proinflammatory or antiinflammatory cytokines, respectively.

Mucus constitutes a physical barrier to the epithelial cells of the intestine and seems to be important in the balanced contact between microorganisms and the host cells of the GI tract. The content of phosphaditylcoline (PC) in the colonic mucus in UC patients is low compared to healthy controls, which possibly weakens the physical barrier. Recently it was shown in a randomized controlled trial (RCT) that 53% of the UC patients were in remission upon a three months treatment with retarded release of PC in the colon (Stremmel et al 2005 Gut 54:966-971).

Irritable Bowel Syndrome (IBS) is part of a spectrum of diseases known as functional gastrointestinal disorders which include diseases such as noncardiac chest pain, nonulcer dyspepsia, and chronic constipation or diarrhea. These diseases are all characterized by chronic or recurrent gastrointestinal symptoms for which no structural or biochemical cause can be found. Patients suffering from IBD and IBS share several kinds of symptoms although it seems that IBS and IBD have no common causes.

In 1907, the inventor of the modern immunology Elias Metchnikoff suggested that some intestinal bacteria have a beneficial role on the health. Today, these bacteria are termed probiotics defined as live microorganisms which administered in adequate amounts confer a beneficial health effect on the host (www.usprobiotics.org). A long range of effects have been postulated for instance that probiotics can help reduce the risk of certain diarrheal illnesses, improve the immune function, reduce the risk of cancer and cardiovascular diseases, assist lactose intolerant people etc. Some of the postulated effects have been investigated scientifically during the last two decades. In particular, research has been intense on the treatment of gastrointestinal diseases using probiotics. The rationale is that changing the microflora in the GI tract of IBD and IBS patients may reduce the immune aggressiveness thereby relieving the symptoms.

In fact, three approaches currently exist for changing the microflora of the intestine including the use of antibiotics, probiotics, and synbiotics. The administration of antibiotics kills a subpopulation of the microflora while probiotics - if administered in appropriate amounts - are thought to displace some of the existing microorganisms in the intestine. Antibiotics and probiotics have also been used in combination. Synbiotics are mixtures of probiotics and substances - socalled prebiotics - that provide a substrate to stimulate the growth of the probiotic microorganisms provided.

Several Lactic Acid Bacteria and species from the genus Bifidobacterium are probiotic, which implies that they one way or another have been shown to promote a specific health effect. Human clinical trials using probiotics alone or in combination with antibiotics have been performed to identify strains and/or formulations for the treatment of patients with IBD or IBS symptoms or for keeping already treated IBD patients in remission. The results from such trials have not been unequivocal since for instance two studies (Nobaek et al. 2000 Am J Gastroenterol. 95:1231-8 and Niedzielin et al. 2001 Eur J Gastroenterol Hepatol. 13:1143-7) using the probiotic strain Lactobacillus plantarum 299v showed some positive effects on IBS patients while another study using the same strain showed no effect on the IBS patients (Sen et al 2002 Dig Dis Sci 47:2615-20). The probiotic bacteria were administered in a daily total amount of up to 2 x 10¹⁰ colony forming units (cfu). Other species of Lactobacillus have been tested as for instance *Lactobacillus rhamnosus* GG, which was administered in an amount of 6 x 10⁹ bacteria twice a day for 52 weeks in a double blinded RCT study for preventing recurrence after curative resection for Crohn's disease (Prantera et al 2002 Gut 51:405-409). This study showed that the probiotic treatment had no effect compared to placebo. Another strategy is to use a combination of different probiotic strains, of which one product is called VSL#3. It consists of eight different bacterial strains and is administered as capsules possibly with freeze or spray dried bacteria. A recent study was performed with the administration to UC patients of 1.8 x 10¹² VSL#3 bacteria twice a day for six weeks (Bibiloni et al 2005 American Journal of Gastroenterology 100:1539-46). The patients had mild to moderate UC symptoms and the study was performed as an open study without placebo. Remission was achieved in 53% of patients, which now has to be confirmed in a placebo controlled trial. In a similar study, 250 mg of the yeast Saccharomyces boulardii was administered three times a day for four weeks. Remission was obtained in 71% of the 17 UC patients (Guslandi et al. 2003 Eur J Gastroenterol Hepatol. 15:697-8). These results are also to be confirmed in controlled studies. A RCT study on maintaining remission of UC was performed recently using the E.coli Nissle strain (Kruis et al. 2004 Gut 53:1617-23). The results showed the bacterium to be as effective as standard 5 ASA therapy.

Several scientific groups and companies each have their own favourite probiotic strain, which is used for molecular analysis, animal studies and/or human clinical trials. Hitherto, the general assumption in the scientific community is that probiotics are promising (Schultz et al. 2003 Dig. Dis. 21:105-28 and Kim et al. 2003 Aliment Pharmacol Ther. 17:895-904), that more studies are needed and that a single strain appropriate for the treatment of a variety of diseases may not be found. Instead, there will be one or more strains for each disease and possibly a variety of strains optimal for each individual patient. WO96/29083 and EP 554418 disclose two intestine colonizing lactobacillus strains including Lactobacillus plantarum 299v (DSM 6595) and Lactobacillus casei ssp. rhamnosus 271 (DSM 6594). EP 415941 discloses methods for preparing nutrient composition comprising treatment of oat gruel with enzymes before mixing with lactobacilli.

In spite of a promising potential, a considerably improvement of the effect of probiotics for use in the treatment of IBD and other gastrointestinal diseases is needed. The improvement of the probiotic effect has been suggested to be found either by i) increasing the number of the administered probiotic microorganisms, ii) combinatorial use of probiotics with antibiotics or the common medicines like 5 ASA or iii) synbiotics. However, none of these methods have proven to provide the desired efficiency.

The number of microorganisms in the GI tract is approximately 10¹⁴ (Bäckhed et al. 2005 Science 307:1915-20). As, described, the results from clinical trials have shown that administration of large amount of probiotics has a positive effect on the IBD symptoms. However, administration to IBD patients of more than 10¹² VSL#3 microorganisms each day for several weeks led to the identification of only small amount of only two of the provided eight strains in biopsies from the patients (Bibiloni et al. 2005 American Journal of Gastroenterology 100:1539-46). No dramatic change in the composition of the microflora was observed.

A novel strategy to considerably improve probiotic treatments is needed to obtain a profound reduction in symptoms in IBD and IBS patients.

### DISCLOSURE OF THE INVENTION

### Summary of the invention

The present invention concerns the use of probiotics and a suitable carrier in a novel treatment strategy that considerably improves the conventional probiotic treatments of IBD and IBS patients. In addition to using probiotic microorganisms, the treatment uses the carrier of the probiotics as an active substance.

The novel concept for the treatment or maintenance treatment of IBD, IBS, gastroenteritis caused by pathogenic microorganisms or other gastrointestinal disorders includes the following
- Two or more daily intakes of an effective amount of fermented cereal composition preferably fermented with probiotic bacteria for example oat gruel fermented with and containing at least 10⁸ colony forming units (cfu) per ml of one or more intestine colonizing Lactobacillus spp. The first intake of fermented cereal composition is preferably before breakfast and the last intake is preferably after the the last meal and drink - except for water - of the day. One intake per day may be sufficient. However, this is not optimal.
- The probiotic bacteria are preferably not freeze or spray dried in the ready-to-use product
- A more efficient treatment or maintenance treatment is obtained if milk products, milk components and easily fermentable sugars such as sucrose, lactose, glucose or fructose are omitted in the fermented product and avoided in the diet.
- The duration of the treatment is dependent on each individual patient and the stage of the disease. The typical treatment periods range from one to 25 weeks but there is no limitation.

Accordingly the present invention concerns the use of an efficient amount of a fermented cereal composition in the preparation of a medicament for the treatment of a gastrointestinal disease such as IBD, IBS, gastroenteritis caused by pathogenic microorganisms or other gastrointestinal disorders.

The composition preferably comprises an efficient amount of non-pathogenic bacteria, which for example may be one or more Lactobacillus spp. and/or Bifidobacterium spp.

The cereal is preferably oat, which is fermented by non-pathogenic bacteria, e.g. one or more Lactobacillus spp.

The present invention also concerns a medicament for the treatment of a gastrointestinal disease, comprising an effective amount of a fermented cereal composition and one or more non-pathogenic bacteria for instance intestine colonizing Lactobacillus spp. or Bifidobacterium spp.

### Detailed description of the invention

IBD is very likely a result of imbalances in the immune system, which may be initiated and preserved by the residing microorganisms of the GI tract including pathogenic or opportunistic pathogenic microorganisms. Attempts to change the flora of microorganisms in the GI tract of IBD or IBS patients have been performed by several scientific groups using probiotic microorganisms and/or antibiotics and/or prebiotics. The strategy has also covered the administration of large amounts of probiotic microorganisms. However, the results from these attempts have been promising but limited both in terms of changing the flora but most importantly the clinical effects.

The present discovery of a correlation between a large intake of fermented oat containing a Lactobacillus strain and the clinical effects as described in Example 2 has successfully been developed into the present invention which offers a surprising novel and advantageous treatment concept for the treatment of IBD and IBS.

The terms used in the present description of the invention are defined below.

The term "probiotic" is defined as "live microbial feed supplement which beneficially affects the host animal by improving its intestinal microbial balance" as defined by G. W. Tannock in "Probiotics: A Critical Review " G. W. Tannock, Ed. Horizon Scientific Press, Wymondham, UK, 1999. ISBN 1-898486-15-8.

The term "animal" includes humans and domestic animals.

The term "a gastrointestinal disease" includes the diseases ulcerative colitis (UC) and Crohn's disease (CD) (collectively termed inflammatory bowel diseases (IBD)), irritable bowel syndrome (IBS), gastroenteritis caused by pathogenic microorganisms and other gastrointestinal disorders.

The term "IBS" is defined as a spectrum of diseases known as functional gastrointestinal disorders which include diseases such as noncardiac chest pain, nonulcer dyspepsia, and chronic constipation or diarrhea. These diseases are all characterized by chronic or recurrent gastrointestinal symptoms for which no structural or biochemical cause can be found.

The term "cereal" is defined as any plant from the grass family that yields an edible grain (seed). The most common grains are barley, corn, millet, oats, quinoa, rice, rye, sorghum, triticale, wheat and rice. Most preferred is oats.

The term "cereal gruel" is defined as cereal grains, flakes, meal, extracts or flour boiled in a liquid, which preferably is water. The cereal gruel is prepared in this way in order to sterilize the suspended cereal and to prepare for the microbial fermentation. By cereal gruel is meant any rheological form of suspended and boiled cereal.

By the term "efficient amount" is to be understood an amount of fermented cereal composition including one or more non-pathogenic bacteria that results in a relatively fast reduction of symptoms in the patient, at least within seven days from the treatment onset. The reduction in symptoms comprises stop of bloody stools and/or a 50 % reduction in the number of daily stools. The amount of cereal used in the preparation of the cereal gruel is commonly known to any person having prepared for example oat gruel. It may also be prepared as disclosed in EP 415941. Oat gruel may be prepared by for example using at least 1 g oats per 100 ml liquid or more preferred 10 g oats per 100 ml liquid or even more preferred at least 20 g of oats per 100 ml liquid or most preferred at least 30 g of oats per 100 ml liquid. The efficient amount of non-pathogenic bacteria in the fermented cereal composition is at least 10⁸ cfu/ml or more preferred at least 10⁹ cfu/ml.

The fermented cereal composition is produced by adding an amount of non-pathogenic bacteria to the cereal gruel resulting in the desired amount of cfu/ml in the fermented cereal composition following fermentation at an optimal temperature for at least 12 hours or preferred at least 24 hours. Prior to the fermentation, the cereal gruel may be treated with enzyme(s) e.g. amylase or/and added a sufficient amount of energy and carbon source for the growth of the selected non-pathogenic bacteria. Prior to or following to the fermentation, the cereal gruel may be treated with enzyme(s) and/or treated physically/mechanically e.g. to reduce the viscosity, to improve the availability of nutrient components, to break down certain cereal molecules, to change the composition of macromolecules and building block molecules, to improve the shelf life or other improvements or modifications. Similarly, the cereal gruel may prior to or following the fermentation be added components to improve or modify the product e.g. by adding lipids, proteins, amino acids and/or fibres that for instance may improve the mucus barrier in patients or may improve the transit time of the food in the GI tract.

The term "fermented cereal composition" is defined as the resulting product from the growth of one or more microorganisms on a cereal gruel and containing one or more non-pathogenic bacteria. Fermented cereal composition is also referred herein as "fermented product" or "fermented oat gruel".

The term "non-pathogenic bacteria" means bacterial strains incapable of causing disease in animals under normal conditions.

By the term "efficient amount" is to be understood an amount of fermented cereal composition including one or more non-pathogenic bacteria that results in a relatively fast reduction of symptoms in the patient at least within seven days from the treatment onset. The reduction in symptoms comprises stop of bloody stools and/or a 50 % reduction in the number of stools per day. The preferred amount of cereal for the preparation of the cereal gruel is for instance at least 1 g cereal per 100 ml liquid or preferably at least 10 g cereal per 100 ml liquid or more preferred at least 20 g of cereal per 100 ml liquid or most preferred at least 30 g of cereal per 100 ml liquid. The efficient amount of non-pathogenic bacteria in the fermented cereal composition is at least 10⁸ cfu/ml, or for example at least 10⁹ cfu/ml.

The fermented cereal composition is produced by adding an amount of non-pathogenic bacteria to the cereal gruel resulting in the desired amount of cfu/ml in the fermented cereal composition following fermentation at an optimal temperature for at least 12 hours or for example at least 24 hours. Prior to the fermentation, the cereal gruel may be treated with enzyme(s) e.g. amylase or/and added a sufficient amount of energy and carbon source for the growth of the selected non-pathogenic bacteria.

The term "Lactobacillus spp. includes any of the following species: Lactobacillus acetotolerans, Lactobacillus acidipiscis, Lactobacillus acidophilus, Lactobacillus agilis, Lactobacillus algidus, Lactobacillus alimentarius, Lactobacillus amylolyticus, Lactobacillus amylophilus, Lactobacillus amylovorus, Lactobacillus animalis, Lactobacillus arizonensis, Lactobacillus aviarius, Lactobacillus bifermentans, Lactobacillus brevis, Lactobacillus buchneri, Lactobacillus casei, Lactobacillus coelohominis, Lactobacillus collinoides, Lactobacillus coryniformis subsp. coryniformis, Lactobacillus coryniformis subsp. torquens, Lactobacillus crispatus, Lactobacillus curvatus, Lactobacillus cypricasei, Lactobacillus delbrueckii subsp. bulgaricus, Lactobacillus delbrueckii subsp delbrueckii, Lactobacillus delbrueckii subsp. lactis, Lactobacillus durianus, Lactobacillus equi, Lactobacillus farciminis, Lactobacillus ferintoshensis, Lactobacillus fermentum, Lactobacillus fornicalis, Lactobacillus fructivorans, Lactobacillus frumenti, Lactobacillus fuchuensis, Lactobacillus gallinarum, Lactobacillus gasseri, Lactobacillus graminis, Lactobacillus hamsteri, Lactobacillus helveticus, Lactobacillus helveticus subsp. jugurti, Lactobacillus heterohiochii, Lactobacillus hilgardii, Lactobacillus homohiochii, Lactobacillus intestinalis, Lactobacillus japonicus, Lactobacillus jensenii, Lactobacillus johnsonii, Lactobacillus kefiri, Lactobacillus kimchii, Lactobacillus kunkeei, Lactobacillus leichmannii, Lactobacillus letivazi, Lactobacillus lindneri, Lactobacillus malefermentans, Lactobacillus mali, Lactobacillus maltaromicus, Lactobacillus manihotivorans, Lactobacillus mindensis, Lactobacillus mucosae, Lactobacillus murinus, Lactobacillus nagelii, Lactobacillus oris, Lactobacillus panis, Lactobacillus pantheri, Lactobacillus parabuchneri, Lactobacillus paracasei subsp. paracasei, Lactobacillus paracasei subsp. pseudoplantarum,, Lactobacillus paracasei subsp. tolerans, Lactobacillus parakefiri, Lactobacillus paralimentarius, Lactobacillus paraplantarum, Lactobacillus pentosus, Lactobacillus perolens, Lactobacillus plantarum, Lactobacillus pontis, Lactobacillus psittaci, Lactobacillus reuteri, Lactobacillus rhamnosus, Lactobacillus ruminis, Lactobacillus sakei, Lactobacillus salivarius, Lactobacillus salivarius subsp. salicinius, Lactobacillus salivarius subsp. salivarius, Lactobacillus sanfranciscensis, Lactobacillus sharpeae, Lactobacillus suebicus, Lactobacillus thermophilus, Lactobacillus thermotolerans, Lactobacillus vaccinostercus, Lactobacillus vaginalis, Lactobacillus versmoldensis, Lactobacillus vitulinus, Lactobacillus vermiforme, Lactobacillus zeae. The preferred species is Lactobacillus plantarum.

The term "Bifidobacterium spp." includes any of the following species: Bifidobacterium adolescentis, Bifidobacterium aerophilum, Bifidobacterium angulatum, Bifidobacterium animalis, Bifidobacterium asteroides, Bifidobacterium bifidum, Bifidobacterium boum, Bifidobacterium breve, Bifidobacterium catenulatum, Bifidobacterium choerinum, Bifidobacterium coryneforme, Bifidobacterium cuniculi, Bifidobacterium dentium, Bifidobacterium gallicum, Bifidobacterium gallinarum, Bifidobacterium indicum, Bifidobacterium longum, Bifidobacterium longum bv Longum, Bifidobacterium longum bv. Infantis, Bifidobacterium longum bv. Suis, Bifidobacterium magnum, Bifidobacterium merycicum, Bifidobacterium minimum, Bifidobacterium pseudocatenulatum, Bifidobacterium pseudolongum, Bifidobacterium pseudolongum subsp. globosum, Bifidobacterium pseudolongum subsp. pseudolongum, Bifidobacterium psychroaerophilum, Bifidobacterium pullorum, Bifidobacterium ruminantium, Bifidobacterium saeculare, Bifidobacterium scardovii, Bifidobacterium subtile, Bifidobacterium thermoacidophilum, Bifidobacterium thermoacidophilum subsp. suis, Bifidobacterium thermophilum, Bifidobacterium urinalis.

The term "intestine colonizing" means that a microbial strain must be present transiently or permanently in the intestine upon one intake of a sufficient amount of cfu. The analysis for intestine colonizing may be carried out for instance by testing for the presence of a specific strain in the faeces on the days and weeks following the intake. Some Lactobacillus spp. may colonize the human intestine for about 14 days.

The term "ready-to-use product" is defined as the product to be used by patients or medical doctors for instance as a drinkable, eatable, anally administrable or tube administrable product. Drinkable products may for instance be a fermented cereal composition contained in a suitable container. Eatable products may for instance be a fermented cereal composition in a solid or semi-solid formfor example mixed with other eatable ingredients not being contrary to the new treatment concept.. Anally administrable products may for instance be in the form of a suppository and tube administrable products may for instance be in a liquid form that is suitable for tube administration via the oral or rectal route.

The term "daily dose of medicament" means the total dose of medicament to be taken by the patient each day in the treatment period.

The term "multiple daily administration" means that the medicament may be administered twice or even more times a day in the treatment period. The reason is to disperse the efficient amount of the daily intake of the fermented cereal composition in order to keep a thorough pressure on the commensal microflora in the intestine throughout the treatment period. The first intake of fermented cereal composition is preferably before breakfast and the last intake preferably after the last meal and drink - except for water - of the day in order to have a dose of fermented cereal composition that are not disturbed during the night by any other intake.

The term "dairy products" means any product that has been produced from mammal milk and "dairy components" means any component derived, purified or extracted from mammal milk or a dairy product.

The term "easily fermentable sugars" means sugars that constitute an easily accessible source of energy and carbon for microorganisms. Examples of easily fermentable sugars are sucrose, lactose, glucose and fructose.

The term "food or medical additives" means any substance that may be added to improve the fermented product in terms of activity towards the gastrointestinal disorder or towards one or more other diseases. Also, "food or medical additives" means any substance that may be added to improve the shelf life, taste, color, or rheological properties of the product. Examples of substances are taste enhancers, colors, pH and osmo-regulators, vitamins, herbs, herbal components, minerals, viscosity regulators, lipids, emulsifiers, lecithins, phosphaditylcholins, glutamine and other amino acids, antioxidants, blood pressure regulators, pain relief substances etc. The skilled person would readily know which additives would be suitable.

### The development of a novel concept for the treatment or maintenance treatment of IBD, IBS, gastroenteritis caused by pathogenic microorganisms and other gastrointestinal disorders

During the development of the novel treatment concept it was speculated how a pronounced clinical effects could be achieved possibly by an efficient change in the microflora of the intestine. Intake of relatively large amounts of appropriate fermented cereal together with probiotics was shown to have an advantageous clinical effect most likely because it is disadvantageous to most of the microorganisms in the GI tract in terms of substrate for growth and/or life maintenance and advantageous to several probiotic bacteria in terms of an appropriate environment for life maintenance. In light of the latter aspect, the applied probiotic microorganisms benefit physiologically if the fermented cereal both constitutes the growth medium for the microorganisms in the production phase and, concomitantly, is the carrier of the microorganisms. Last but not least, cereals contain a range of components important to man such as carbohydrates, fibres and lipids. Cereals like oats and maize contain relatively large amounts of lipids. Recently, it was shown that intake of phosphatidylcholine (lecithin) - a polar lipid - improves the condition for ulcerative colitis patients apparently by providing material for the generation of a sufficient mucus layer in the GI tract (Stremmel et al. 2005 Gut 54:966-971). It may be speculated that a high amount of polar lipids in cereals may be co-responsible for the positive effects of the novel treatment protocol described here. Accordingly cereals with a high amount of polar lipids, such as oat, might be preferred.

According to the strategy on efficiently changing the microflora in the GI tract, intake of components that stimulate the growth and maintenance of the residing microflora should be avoided. Most milk components and sugars like sucrose, lactose, glucose or fructose are easily utilized by bacteria such as E. coli. For instance, in competition experiments in flasks most E. coli strains would be much faster in utilizing milk components and the sugars mentioned than most lactic acid bacteria or Bifidobacterium strains. Therefore, milk products, milk components and easily fermentable sugars should be omitted in the present fermented cereal composition and in the treatment or maintenance treatment concept.

The applied probiotic bacteria should preferably be able - at least transiently - to reside in or colonize the GI tract. Also, the probiotic microorganisms should be physiologically robust. There is a long range of probiotic products on the market and many contain freeze or sprayed dried probiotic microorganisms (DDS-Plus® (UAS Lab, MN, USA), VSL#3 (Sigma-Tau, MD, USA), Lp299v (Quest, UK) etc.). Freeze or spray drying usually kills a large percentage of the microorganisms and severely stresses the surviving population (Wang et al. 2004 Int J Food Microbiol. 93:209-17). Although pretreatment of several microorganisms under mild stress conditions may be beneficial for the survival under future harsh conditions - for instance in the GI tract - , freeze or spray drying is most likely too rough to result in probiotic microorganisms that are physiologically robust.

One example of the novel concept for the treatment or maintenance treatment of IBD, IBS, gastroenteritis caused by pathogenic microorganisms or other gastrointestinal disorders includes the following
- Two or more daily intakes of an effective amount of composition)) oat gruel fermented with and containing about 10⁸ - 10⁹ cfu/ml of one or more intestine colonizing Lactobacillus spp. The first intake of fermented oat gruel is performed before breakfast and the last intake preferably performed as the last meal and drink - except for water - of the day.
- The probiotic bacteria are preferably not freeze or spray dried in the ready-to-use product
- A more efficient treatment or maintenance treatment is obtained if milk products, milk components and easily fermentable sugars such as sucrose, lactose, glucose or fructose are omitted in the fermented product and avoided in the diet.
- The duration of the treatment is dependent on each individual patient and the stage of the disease. The typical treatment periods range from one to 25 weeks but there is no limitation.

The probiotics may be any non-pathogenic microorganisms preferably lactic acid bacteria and/or species from the genus of Bifidobacterium and more preferably capable of at least transiently colonizing the intestine.

The carrier is a sufficient amount of fermented cereals, which for example is fermented oat gruel. There are two reasons for administering the fermented cereal. The first is to present an environment in the GI tract in which the properties for growth or life maintenance for many microorganisms are unfavourable except for selected microorganisms including probiotics. The second reason is to provide complex lipids, carbohydrates, proteins, fibres and other molecules that may have a positive effect both in providing material for production of physical barriers such as the mucus layer and on the immune system response. The complex lipids, carbohydrates, proteins and other molecules both originate from the oat and probiotics per se but are also an outcome of the fermentation process.

Subsequent to the fermentation process, the probiotic microorganisms do not grow further in the fermented cereal. Accordingly, the fermented cereal does not serve as a prebiotic compound since it does not stimulate growth of the probiotics. The resulting fermented cereal may have a pH below 5.5, which both stresses and prepares the probiotic microorganism for upcoming stress conditions, which for instance will follow in the stomach and in the intestine of GI patients. The pre-stress treatment in the production of probiotics is a matter of a fine balance. Most probiotic products are available as freeze or spray dried preparations. Freeze or spray drying is known as being extremely rough, which in most cases kills a great deal of the population. The surviving microorganisms may need time for adaptation when presented to a fluid environment. In other words, freeze or spray dried probiotics may not be prepared adequately for robustness to passage of the stomach and/or competing for the colonization of the intestine. However, it may be possible in certain situations to add freeze or spray dried probiotics to the fermented cereal.

The novel treatment described herein may be initiated at any stage of the IBD or IBS course. The treatment should be carried out by following the novel treatment protocol accurately and the duration may vary from one or few weeks to several months. Following a gradual stop in the intake of the fermented cereal composition, IBD patients will usually experience that the IBD symptoms are absent and not reoccurring. However, some mild symptoms may reoccur usually within months or years. These symptoms may be treated by entering a maintenance treatment just by following the novel treatment protocol for a shorter duration, which may be of one or more weeks. Also, IBD and IBS patients may acquire experience telling that symptoms may turn up under specific circumstances for instance at specific times at the year, under certain stress conditions or associated to diets in certain communities. In such cases, patients may benefit from entering a maintenance treatment before meeting the specific circumstances. The duration of this maintenance treatment may also be relatively short e.g. one or more weeks.

In the novel treatment both milk, milk components and easily fermentable sugars are omitted in the fermented cereal composition and avoided in the diet to create an even more unfavourable environment for microbial growth or life maintenance in the GI tract. Foods that contain relatively large amounts of sugar and/or milk components should be avoided. The foods cover sugar, soft drinks (containing sugar), juice, syrups, candy, cakes, cookies, dried fruit, muesli and other breakfast product with added sugar, ice cream, milk, cream, fermented milks, butter, etc. However, a daily intake of up to 1 L of diet sodas without any sugar or milk components added does not exert a negative effect on the treatment. The patients are recommended to take a supplement of calcium since milk and milk components should be avoided in the treatment. However, many calcium tabs contain relatively large amounts of lactose. Therefore, calcium tabs without lactose should be administered. This is an example of unexpected added components in supplement and foods, which the patients must pay attention to. Foods containing traces of milk or milk components and/or very small amounts of added sugar may be accepted in the diet when following the novel treatment concept. However, no exact limits may be stated for the concentration of added milk, milk components and/or sugar in the foods due to the complexity of different foods and each individual food intake.

The terms "novel treatment", "novel treatment strategy" and "novel treatment concept" refer to the treatment of IBD, IBS, gastroenteritis caused by pathogenic microorganisms or other gastrointestinal disorders using the herein described invention.

### EXAMPLES

### Example 1. Conventional probiotic treatment of a 66 years old male patient suffering from a severe IBS did not have any positive effects.

A 66 years old man was suffering from abdominal pain and diarrhea. He was examined by a physician who could not set up an accurate diagnosis. The patient was consequently redirected to a department of gastroenterology at the local hospital. In the meantime, the symptoms were getting worse and at this time the patient lost more than 2 kg in weight per week. The patient underwent a colonoscopy. No signs of ulcers or necrosis were detected in the lower intestine and the colon. The most likely diagnosis was IBS. Following, the patient was redirected to a local gastroenterologist. On top of the described symptoms, occasional paralysis in the patient's legs began to show up.

The patient undertook a treatment based on a protocol that uses probiotics for the treatment of IBS including abdominal pain and diarrhea. The product named ProViva (Skånemejerier, Malmö, SE) was selected as the source of probiotics. In the manufacturer's description in WO96/29083 and in the scientific literature (Niedzielin et al. 2001 Eur J Gastroenterol Hepatol. 13:1143-7) it is described that 200 ml ProViva fruit drink containing 5 x 10⁷ probiotic bacteria per mL should be administered twice each day for four weeks to treat gastrointestinal diseases. Accordingly, the patient took twice a day 10 mL of another ProViva product (Skanemejerier, Malmö, SE) that contains 10⁹ cfu/ml of Lactobacillus plantarum 299v in an oat gruel without added fruit. This amount was chosen to match the number of cfu in Niedzielin et al. 2001. The 10 ml was taken before breakfast and two hours after the last meal in the evening. Having followed this conventional protocol for two weeks, the patient did not experience any positive effect. At that time, the patient had lost more than 30 kg in weight during the three months period from the onset of the disease.

### Example 2. Successful probiotic treatment of a 66 years old male patient suffering from a severe IBS using a novel treatment strategy

Due to the lack of any positive effects from the conventional probiotic treatment, the patient mentioned in Example 1 initiated the novel treatment according to the present invention.

The patient had a daily intake of 500 ml of a fermented oat gruel containing 10⁹/ml Lactobacillus plantarum 299v (Skånemejerier, Malmö, SE). The fermented product was divided into two portions, which were taken each day before breakfast and as the last meal or drink of the day. The patient experienced a significant reduction of symptoms in less than one week following initiation of the novel treatment. A complete remission was obtained by following the novel treatment for eight weeks. One year later the patient had gained the 30 kg in weight and no relapse has occurred until now, which is about five years subsequent to completion of the novel treatment.

### Example 3. Successful treatment of a 19 years old woman with a severe ulcerative colitis using the novel treatment strategy

A colonoscopy revealed an ulcerative colitis in a 19 years old woman. She was extremely tired and had abdominal pain and bloody diarrheas with more than ten defecations a day. She was hospitalized for 14 days and treated with prednisolon (cortisone) and 5 ASA. This treatment was continued when the patient was sent home. 18 weeks later the tiredness and abdominal pain were still present. The diarrhea was also present but the number of defecations per day was reduced to an average of five. Blood was still present in the stools. The patient experienced severe side effects from the prednisolon including a reduced immune status resulting in continuous infections. She then decided to finish the intake of prednisolon by a gradual reduction. The intake of 5 ASA was continued.

One week following the termination of prednisolon intake, the patient started to follow the novel treatment using a fermented oat gruel containing 10⁹/ml Lactobacillus plantarum 299v (Skånemejerier, Malmö, SE) as described in Example 2. The intake of 5 ASA was continued in parallel for 12 weeks. One week after the treatment start, blood was absent from the stools. On day No. 4 in the novel treatment, the patient's tiredness disappeared. In the following three weeks, the number of defecations varied from two to four. Abdominal pain occurred occasionally during the first three weeks in the novel treatment. During weeks Nos. 4-16, the number of defecations was one or two. The patient experienced two courses of abdominal pain during this period. These were characterized as short pain courses similar to uncomplicated incidents experienced by the patient in the childhood. The treatment was stopped 16 weeks after initiation by a gradual reduction in the daily intake of the fermented oat gruel containing probiotics.

The patient had no UC symptoms throughout the following three and a half year. Subsequently, however, a relapse occurred with a relatively mild diarrhea. The patient then followed the novel treatment protocol for another three weeks resulting in a remission that has continued since, which is 18 months after completion of the second course of the novel treatment.

### Example 4. Treatment of IBD patients using the novel concept for the treatment of IBD and IBS

A series of treatments was set up during a period of four and a half years subsequent to the successful treatment of the 19 years old UC patient as described in Example No. 3. Additional nine UC or CD patients were enrolled to follow the novel treatment concept. Data, results and comments from the treatments are given in Table 1.

**Table 1. Data, results and comments concerning nine IBD patients enrolled for the novel treatment concept**

| **Sex and age (years)** | **Diagnosis*** | **Treatment period (weeks)** | **Result** | **Comments** |
|---|---|---|---|---|
| Male, 20 | CD, moderate | Two | Partial remission | The patient stopped the treatment twice after two weeks in each course when the symptoms disappeared |
| Female, 26 | UC, moderate | 14 | Remission | Subsequently, the patient has once a year - for two years - followed a maintenance treatment for three weeks. |
| Female 16 | UC, moderate | 16 | Remission | |
| Female, 35 | UC, moderate | Eight | Remission | |
| Female, 17 | CD, moderate | Four** | Remission | Currently in treatment. |
| Female, 45 | UC, mild | Three | Remission | |
| Female, 19 | CD, moderate | <One | No effect | Drop out. The patient apparently did not initiate the treatment |
| Female, 16 | UC, severe | One | Positive response | Bleeding stopped and diarrhea significantly reduced during the one week treatment. |
| Male, 10 | UC, severe | Five** | Remission | Currently in treatment. Bleeding stopped and a significant reduction of diarrheas during the first week. Two defecations from the end of the second week. Occasional abdominal pain during the first four weeks. |

| | | | | |
|---|---|---|---|---|
| * UC: Ulcerative colitis; CD: Crohn's disease. The stage of the disease was judged prior to treatment start and stated as mild (below 3 in SCCAI), moderate (about 4-7 in SCCAI) or severe (about more than 8 in SCCAI). ** Treatment period until now. Currently in treatment | | | | |

Every patient - except for one drop-out - responded positively to the novel treatment concept. Remission was achieved in the cases where the patients followed the treatment protocol accurately. The results strongly indicate that the novel treatment is capable of removing the symptoms from UC and CD patients regardless of the stage of the disease. A requirement for a successful treatment is to follow the treatment protocol accurately and continue the treatment for an appropriate duration (Table 1).

### Example 5. Treatment of IBS patients using the novel treatment concept

One woman with IBS and three women with GI disorders followed the novel treatment protocol for two or three weeks. The first experienced diarrhea and abdominal pain while the three had relatively mild or moderate diarrheas. Data and results from the treatments are given in Table 2.

**Table 2. Data and results concerning four female IBS or GI disorder patients that followed the novel treatment concept**

| **Sex and age (years)** | **Diagnosis*** | **Treatment period (weeks)** | **Result** |
|---|---|---|---|
| Female, 20 | IBS | Three | Remission |
| Female, 47 | GI disorder | Three | Remission |
| Female, 49 | GI disorder | Two | Remission |
| Female, 72 | GI disorder | Three | Remission |

| | | | |
|---|---|---|---|
| * IBS described as severe symptoms while GI disorder described as mild to moderate symptoms | | | |

The treated patients all achieved remission. Despite the limited number of patient, the results strongly indicate that the novel treatment concept is capable of turning IBS and/or GI disorders into remission.

### Example 6. Maintenance treatment of IBS and IBD patients using the novel treatment concept

Three women who had previously completed a successful treatment of either UC (Table 1) or GI disorders (Table 2) followed the treatment protocol for maintenance reasons. Data, results and comments from the treatments are shown in Table 3.

**Table 3. Data, results and comments concerning three female patients that followed the novel treatment concept for maintenance**

| **Sex and age (years)** | **Diagnosis*** | **Treatment period (weeks)** | **Result** | **Comments** |
|---|---|---|---|---|
| Female, 19 | UC, medium | 2 x three | No UC symptoms | The patient has once a year - for two years - followed the maintenance treatment for three weeks. |
| Female, 47 | GI disorder | > 20 | No GI disorder symptoms | Previously experienced symptoms have not been present since initiation of the maintenance treatment |
| Female, 72 | GI disorder | > 20 | No GI disorder symptoms | Previously experienced symptoms have not been present since initiation of the maintenance treatment |

The three women have not experienced any symptom from their UC or GI disorders since the initiation of the maintenance treatment. The results strongly indicate that the novel treatment concept is efficient in the maintenance treatment of UC and GI disorders despite the low number of patients

### Example 7. Description of a RCT study on patients suffering from ulcerative colitis using the novel concept for the treatment of IBD and IBS

In order to supplement the results described in the previous examples, a randomized clinical trials (RCT) is planed. A Phase II, double blinded placebo RCT study will be performed on UC or CD patients essentially as described by for instance by Stremmel et al. 2005 Gut, 54: 966-971. The novel treatment protocol will include the use of fermented oat gruel, which has been fermented with and contains L. plantarum 299v and/or similar non-pathogenic bacteria. The amount of intake in the morning and the evening may be fixed at for instance 250 ml fermented oat gruel containing 10⁹cfu/ml. The placebo may be cellulose beads in a water solution in which the pH has been adjusted to about 5 with lactic acid and/or acetic acid to mimic the active product, or any other appropriate placebo means. The trial may for instance run for a period between three and 20 weeks depending on the stage of the enrolled UC or CD patients. One expected end point would be remission of at least 50% of the patient to CDAI values ≤ 2 within three or four weeks.

## Claims

1. Use of an efficient amount of a fermented cereal composition in the preparation of a medicament for the treatment of a gastrointestinal disease.

2. Use according to claim 1, wherein the composition comprises an efficient amount of non-pathogenic bacteria.

3. Use according to claim 2, wherein the non-pathogenic bacteria are selected from one or more Lactobacillus spp. and/or Bifidobacterium spp.

4. Use according to claim 2, wherein the composition comprises an efficient amount of one or more non-pathogenic intestine colonizing Lactobacillus spp.

5. Use according to any of the claims 1-4, wherein the cereal is oat.

6. Use according to any of the claims 1-5, wherein the cereal is fermented by one or more Lactobacillus spp.

7. Use according to any of the claims 2 to 6, wherein the composition comprises at least 10⁸ colony forming units (cfu) of bacteria/ml.

8. Use according to claim 7, wherein the composition comprises at least 10⁹ colony forming units (cfu) of bacteria/ml.

9. Use according to any of claims 2-8, wherein the applied bacteria are not freeze or spray dried in the ready-to-use product.

10. Use according to any of the claims 1 to 9, wherein the gastrointestinal disease is any of the diseases Crohn's disease, ulcerative colitis, Irritable Bowel Syndrome (IBS) or any other gastrointestinal disorder.

11. A medicament for the treatment of a gastrointestinal disease, comprising an effective amount of a fermented cereal composition and non-pathogenic bacteria, wherein the daily dose of medicament comprises at least 5x10¹⁰ of the non-pathogenic bacteria.

12. A medicament for the treatment of a gastrointestinal disease, comprising an effective amount of a fermented cereal composition and Lactobacillus spp. and/or Bifidobacterium spp, wherein the daily dose of medicament comprises at least 5x10¹⁰ of the Lactobacillus spp. and/or Bifidobacterium spp.

13. A medicament for the treatment of a gastrointestinal disease, comprising an effective amount of a fermented cereal composition and one or more non-pathogenic intestine colonizing Lactobacillus spp, wherein the daily dose of medicament comprises at least 5x10¹⁰ of the one or more non-pathogenic intestine colonizing Lactobacillus.

14. A medicament according to claims 11-13, wherein the gastrointestinal disease is any of the diseases Crohn's disease, ulcerative colitis, IBS or any other gastrointestinal disorder.

15. A medicament according to claims 11-14, wherein the cereal is oat.

16. A medicament according to any of the claims 11-15 in a form suitable for multiple daily administrations.

17. A medicament according to any of the claims 11-16, wherein said medicament essentially does not contain any dairy products, dairy components or any easily fermentable sugars such as sucrose, lactose, glucose or fructose.

18. A medicament according to any of the claims 11-17, further comprising food or medical additives such as taste enhancers, colors, pH and osmo-regulators, vitamins, herbs, herbal components, minerals, viscosity regulators, lipids, emulsifiers, lecithins, phosphaditylcholins, glutamine and other amino acids, antioxidants, blood pressure regulators, pain relief substances.

19. A medicament according to any of the claims 11-18, further comprising an additional active agent suitable for treatment of gastrointestinal disorders or other diseases.

20. A medicament according to any of the claims 11-19, in the form of a drinkable, eatable, anally administrable or tube administrable product.
